# EUROPEAN PATENT APPLICATION

(11) **EP 4 364 686 A1**
(43) Date of publication of application: **08.05.2024**
(21) Application number: 23206966.6
(22) Date of filing: 31.10.2023
(51) Int. Cl.: A61B 34/20, A61C 1/08, A61F 2/28, A61B 34/30, A61B 34/10, A61B 90/00

(54) **MOTION TRACKING SYSTEM FOR DENTAL IMPLANTATION**

(30) Priority: 01.11.2022 KR 20220143461
(71) Applicant: Dentium Co., Ltd., Gyeonggi-do 16229 (KR)
(72) Inventor: Lee, In Jae, 16229 Suwon-si, Gyeonggi-do (KR); Sung, Mun Hyun, 16229 Suwon-si, Gyeonggi-do (KR); Choi, Young Hee, 16507 Yeongtong-gu, Suwon-si (KR); Heo, Jun Mu, 17056 Cheoin-gu, Yongin-si (KR); Kim, Min Ji, 16509 Yeongtong-gu, Suwon-si (KR); Jung, Won Jae, 16705 Yeongtong-gu, Suwon-si (KR)
(74) Representative: von Bülow & Tamada

(57) **Abstract**

A motion tracking system for dental implantation related to the present invention includes: a base that is formed to generate an electromagnetic field; a detection unit that is attached around a procedure target site and includes a sensor detecting the electromagnetic field generated from the base; and a control unit that receives a measurement signal from the sensor, calculates a variation in a position and direction of the procedure target site, and outputs the calculated variation through a display, in which the detection unit is formed to be attached to teeth spaced from the procedure target site in a dental arch of a patient using an adhesive means, and is classified into a plurality of types depending on the position or direction of the sensor.

## Description

### BACKGROUND

### 1. FIELD

The present invention relates to a motion tracking system for dental implantation.

### 2. DESCRIPTION OF RELATED ART

As we enter an aging society in earnest, interest in medical technologies that can solve maintenance, treatment, or the like of health is increasing. In addition to orthopedic surgery closely related to bone health which is gaining prominence as we enter an aging era, dental implantation is also increasing.

The dental implants are artificial teeth that are placed into a gum bone and are used when there are no teeth or the surrounding teeth are weak.

In the dental implantation, it is difficult to guarantee a high degree of accuracy because a generation operation of a hole into which a fixture will be inserted relies on a doctor's sense of hand. To compensate for this, in order to accurately place implants, a guide device such as a surgical guide that guides a section requiring drilling and fixture placement have appeared. However, since the guide device covers the entire dental arch, patients subjected to dental implantation may feel uncomfortable, and the resources and time required to manufacture the guide are significant. In addition, an operator should maintain a posture that puts strain on a neck and back of a patient in order to check an oral condition of the patient, so not only does it make the operator uncomfortable, but it may also become difficult to ensure accuracy during the continuous procedure.

### SUMMARY

The present invention provides a motion tracking system for dental implantation capable of performing real-time motion tracking without providing an implant guide device for each patient.

In addition, the present invention is to utilize a sensor installed in an oral cavity that can detect electromagnetic fields to eliminate patient discomfort due to its compact size and minimize interference during procedure due to a direction or arrangement of the sensor, and acquire real-time patient motion information to increase the accuracy of the procedure.

The problems of the present invention are not limited to those mentioned above, and other technical problems will be clearly understood by those skilled in the art from the following description.

According to an aspect of the present invention, a motion tracking system for dental implantation includes: a base that is formed to generate an electromagnetic field; a detection unit that is attached around a procedure target site and includes a sensor detecting the electromagnetic field generated from the base; and a control unit that receives a measurement signal from the sensor, calculates a variation in a position and direction of the procedure target site, and outputs the calculated amount of change through a display, in which the detection unit may be formed to be attached to teeth spaced from the procedure target site in a dental arch of a patient of using an adhesive means, and may be classified into a plurality of types depending on the position or direction of the sensor.

The detection unit may include a jig that has an inner side surface surrounding the teeth and outer side surface formed to have the sensor arranged thereon.

The jig may include a first side wall part, a second side wall part, and a head part connecting between the first side wall part and the second side wall part, and the sensor may be positioned in at least one selected from the first side wall part, the second side wall part, and the head part depending on the type of the detection unit.

At least one of the first side wall part and the second side wall part may include a slot through which the adhesive means seeps out.

The motion tracking system may further include reinforcement ribs that are formed along edges of the first side wall part and the second side wall part.

The motion tracking system may further include a plurality of through holes that are formed in the head part and formed to allow the adhesive means to seep out.

The type of the detection unit may include a form in which the first side wall part and the second side wall part are parallel and is not parallel.

The jig may be made of a non-magnetic material.

The sensor may include a housing that is formed to be directly attached to teeth by the adhesive means.

The adhesive means may include putty containing an adhesive.

The motion tracking system may further include a computed tomography device that is configured to image the procedure target site and the detection unit, in which the sensor may include a plurality of metal beads having magnetism arranged so that the sensor position is detected in an image captured by the computed tomography device.

The control unit may be configured to reflect and output the variation in the position and direction of the procedure target site calculated through the measurement signal of the sensor when a motion of a patient occurs in a position and direction of an implant planned for the computed tomography image while the detection unit is fixed.

The control unit may be formed to register a procedure plan with a second image based on the detection unit from a first image obtained by computed tomography while the detection unit is fixed, a procedure plan including a position and direction of an implant to be placed in the first image, and the second image obtained by the computed tomography while the detection unit is fixed after the procedure, and provide a comparison result of the implant through the display after the procedure included in the second image and the procedure plan.

The motion tracking system may further include: a robot that is formed to be operated by a control signal of the control unit according to a procedure plan based on a calculation result of the control unit; and a hole guide that is formed to be detachable from the robot and formed to guide a position and direction of a procedure tool for the procedure target site.

The hole guide may include a mounting part that is mounted on an end effector of the robot; and a guide part that is formed at an end portion of the mounting part and formed with a hole.

The guide part may be formed in a form of a sleeve to guide an entry direction of procedure tools of an operator.

The motion tracking system may further include a body part that is provided with a driving element generating the electromagnetic field; a tool mounting part that is formed to enable a tool to be mounted on a tip of the body part; and a sensor unit that is formed to detect a position of the tool arranged in space to track the position of the tool; and a spacing jig that has one end formed with a support part for fixing the sensor unit and the other end formed with a coupling part coupled to the body part, and is formed to maintain the sensor unit spatially spaced from the driving element.

The spacing jig may include a space extension part connecting the support part and the coupling part.

The body part may extend in a first axis X1 direction, and the tool may be formed to face in a second axis X2 direction, and the sensor unit may be arranged on a plane formed by the first axis X1 and the second axis X2.

The coupling part may be coupled to the body part in a rotated state by a selected angle with respect to the body part so that the sensor unit forms a predetermined angle with respect to the plane formed by the first axis X1 and the second axis X2.

The coupling part may be formed in a form of a ring that is inserted into the body part.

The space extension part may have a direction and length so that the sensor unit is spaced apart from the driving element along the first axis X1 direction by a first offset d1 and is spaced apart from the first axis X1 by a second offset d2.

The motion tracking system may further include a chair part that is formed to adjust a posture of the patient; a chair base that supports the chair part; a robot that is formed to assist procedure by moving an arm with respect to the procedure target site of the patient based on the position; a robot base that is formed to support the robot; and a connection part that connects the chair base and the robot base to integrate the chair portion and the robot.

The chair base may include a plurality of first ground parts for being supported on a ground, and the robot base may include a plurality of second ground parts for being supported on the ground.

The motion tracking system may further include a first spacer that is arranged between the robot and the robot base.

The display may be supported by a balance arm that has one end fixed to the robot base and the other end coupled to the display so that the display changes its position and posture.

The balance arm may be supported by at least one second spacer.

### BRIEF DESCRIPTION OF DRAWINGS

FIG. 1 is a diagram conceptually illustrating a motion tracking system for dental implantation related to the present invention.
FIG. 2 is a schematic perspective view of a sensor related to the present invention.
FIGS. 3 and 4 are diagrams for describing an arrangement of beads installed in the sensor of FIG. 2, and FIG. 3 is a schematic plan view of the sensor, and FIG. 4 is a schematic front view of the sensor.
FIG. 5 is a perspective view illustrating a first type of detection unit related to the present invention.
FIG. 6 is a perspective view of the detection unit of FIG. 5 viewed from top.
FIG. 7 is a perspective view of the detection unit of FIG. 5 viewed from bottom.
FIG. 8 is a perspective view illustrating a second type of detection unit related to the present invention.
FIG. 9 is a perspective view of the detection unit of FIG. 8 viewed from top.
FIG. 10 is a perspective view of the detection unit of FIG. 8 viewed from bottom.
FIG. 11 is a flowchart for describing a method of analyzing implant placement results using the motion tracking system for dental implantation related to the present invention.
FIG. 12 is a diagram schematically illustrating those displayed on a computed tomography image by comparing an appearance before and after implant placement.
FIGS. 13 and 14 are diagrams illustrating an appearance of an implant before and after placement for displaying measurement targets for each analysis type in relation to the present invention.
FIGS. 15 and 16 are screens illustrating before and after placement displayed on the display, respectively.
FIG. 17 is a diagram schematically illustrating a side view of a hole guide related to the present invention.
FIGS. 18A to 18C are plan views illustrating the hole guide of FIG. 17 as an example.
FIG. 19 is a diagram schematically illustrating an appearance in which a hole guide 340 is attached to an end effector 331 of a robot.
FIG. 20 is a flowchart for describing a robot guide method.
FIG. 21 is a diagram conceptually illustrating the motion tracking system for dental implantation to which a handpiece device capable of tracking a position is applied according to an example related to the present invention.
FIG. 22 is a perspective view of the handpiece device capable of tracking the position related to the present invention.
FIG. 23 is an exploded perspective view of the handpiece device capable of tracking the position of FIG. 22.
FIG. 24 is a perspective view of an integrated dental chair system related to the present invention.
FIG. 25 is a perspective view illustrating the dental chair system of FIG. 24 viewed from another side.
FIG. 26 is a perspective view illustrating the dental chair system of FIG. 24 viewed from bottom.
FIG. 27 is a perspective view of the dental chair system according to another example related to the present invention.

### DETAILED DESCRIPTION

Hereinafter, a motion tracking system for dental implantation related to the present invention will be described in detail with reference to the attached drawings. Terms used in the specification and claims may not be limited to dictionary meanings, and may be appropriately defined and understood to explain the present invention in the best way.

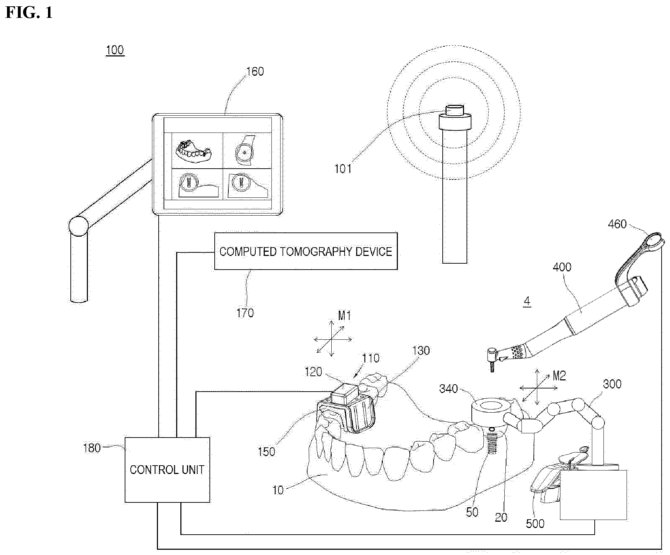

Referring to FIG. 1, a motion tracking system 100 for dental implantation related to the present invention includes elements that tracks a motion of a patient 10 or a change in a position or direction of the procedure target site 20 in real time while performing the dental implantation using a procedure tool 4 such as a handpiece 400 with respect to a procedure target site 20 of the patient 10, and provides the tracked motion and change to an operator, etc., to enable accurate and convenient procedure. Examples of these elements include a base 101, a detection unit 110 having a sensor 120 and a jig 130, a display 160, a computed tomography device 170, a control unit 180, etc.

The dental implantation includes processes of drilling a hole in an alveolar bone using the handpiece 400 according to a certain sequence and inserting an implant 50 into the procedure target site 20 according to a procedure plan. The computed tomography device 170 is used to check the patient 10 and the procedure target site 20 and establish the procedure plan before placement of the implant, and check analysis results in comparison with the procedure plan after the procedure as described later.

In order to detect that the motion of the patient 10 and the change in the position or direction of the procedure target site 20 during the procedure may be detected, the motion tracking system 100 for dental implantation includes the base 101 that generates an electromagnetic field (EM) and the detection unit 110 for detecting the electromagnetic field of the base 101. The base 101 may be installed within a radius (e.g., 750 mm) of a level at which the motion of the patient 10 may be detected. The detection unit 110 is attached around the procedure target site 20 in an oral cavity of the patient 10 and has a sensor 120 that detects the electromagnetic field generated from the base 101. In order to firmly fix the sensor 120 in the oral cavity of the patient 10, the sensor 120 may be fixed to teeth by the jig 130 (FIG. 1), or the sensor 120 may be directly fixed to the teeth. The detection unit 110 is attached, by an adhesive means 150, to teeth spaced at a certain distance from the procedure target site 20 in a dental arch of the patient 10. In this case, a certain distance is a section that requires drilling of the handpiece 400 during implantation, and may be set to a distance that does not interfere with procedure in consideration of a procedure process of an operator.

A display 160 is installed to provide various visual information or procedure situations during the procedure. In addition, the present system 100 includes a control unit 180 that receives a measurement signal from the sensor 120, calculates the variation in the position and direction of the procedure target site 20, and outputs the calculated amount of change through the display 160. The control unit 180 is configured to receive a signal (POS) from the sensor 120 and provide a calculation result of the variation in movement M1 of the sensor 120 in the form of X, Y, Z, roll, pitch, and yaw values. The calculation result of the control unit 180 is presented through the display 160. For example, the calculation result uses a result of a registration process with a reference value of the sensor 120 to reflect the variation in the position and direction calculated in real time in the position and direction of the implant planned through the computed tomography image.

By using the motion information of the patient 10 secured in this way, the motion tracking system 100 for dental implantation may accurately guide the position of dental implantation to an operator using guide software or programs.

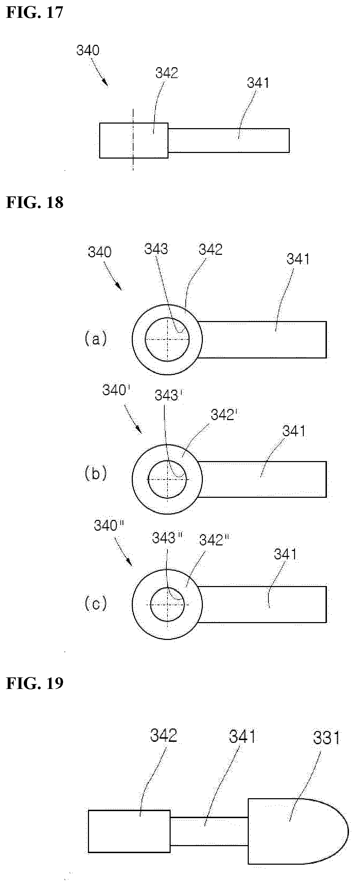

As one embodiment according to the present invention, implant guiding may be implemented in the motion tracking system for dental implantation by using a robot 300 and a hole guide 340. This will be described below with reference to FIGS. 17 to 20.

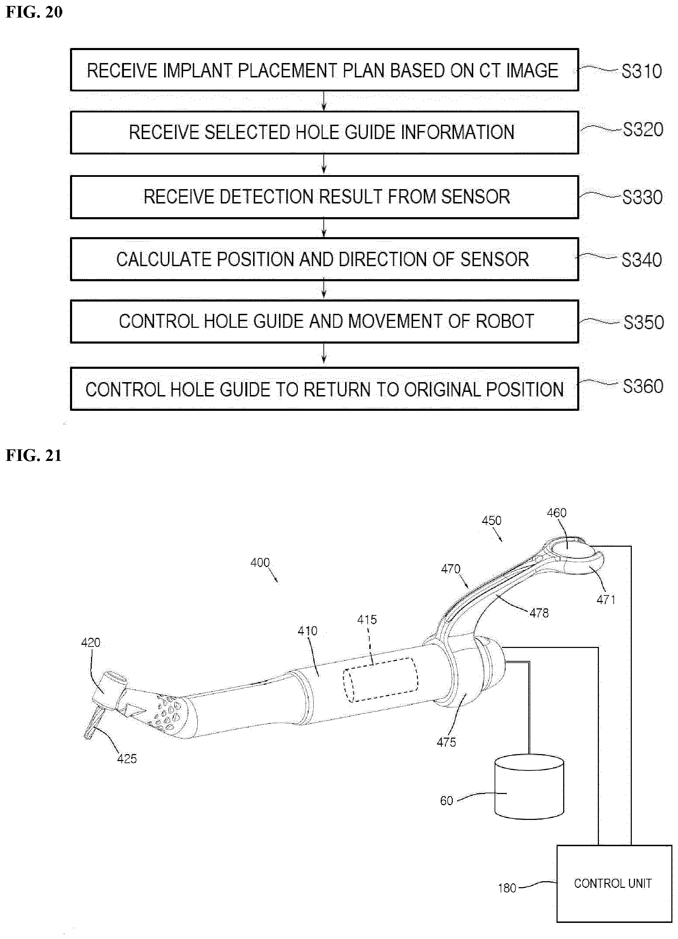

In addition, as another embodiment according to the present invention, a handpiece device 400 capable of tracking movement M2 may be applied to the motion tracking system for dental implantation. This will be described below with reference to FIGS. 21 to 23.

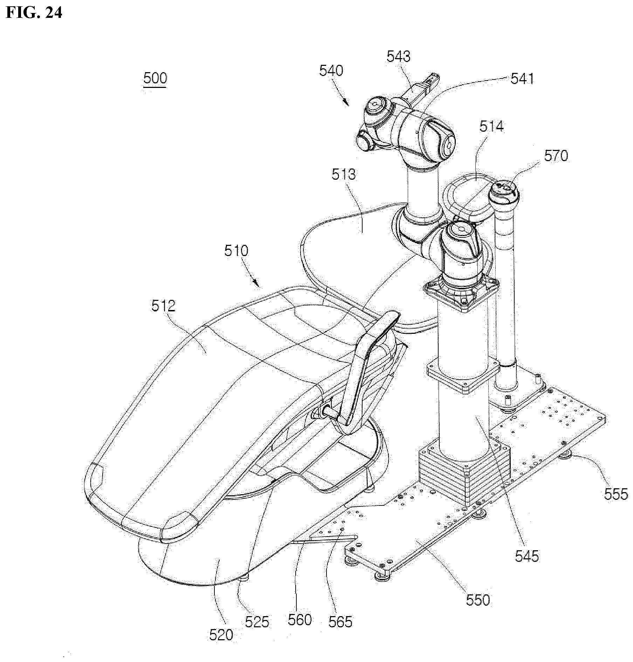

In addition, as another embodiment according to the present invention, an integrated chair unit system 500 may be applied to the motion tracking system for dental implantation. This will be described below with reference to FIGS. 24 to 27.

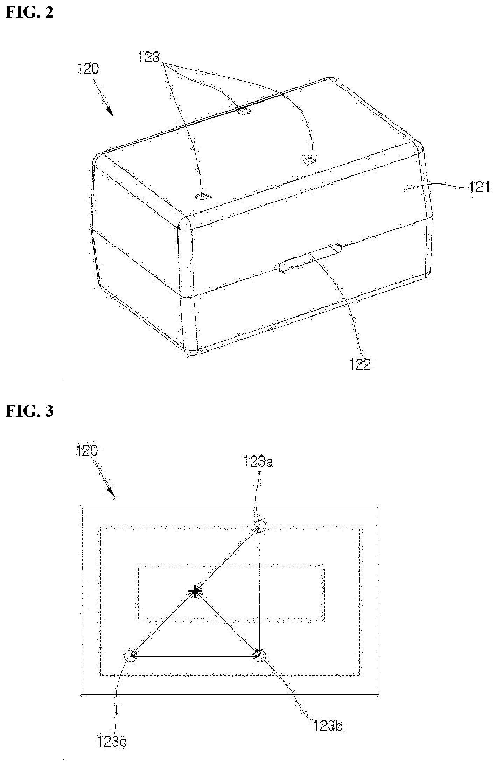

FIGS. 2 to 4 illustrate a sensor 120 related to the present invention. The sensor 120 may be surrounded by a housing 121 having a cable connection part 122 for electrical connection to the control unit 180. A circuit board 124 on which a magnetic sensor 125 is mounted is installed inside the housing 121.

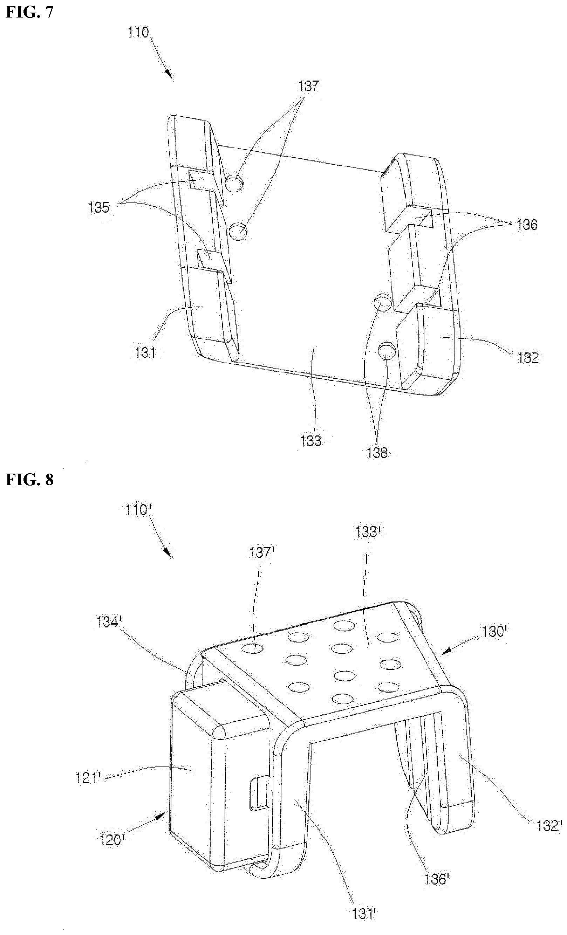

An outside of the housing 121 is provided with a plurality of beads 123, 123a, 123b, and 123c that may provide an image that may be distinguished from a living body during computed tomography. The beads 123, 123a, 123b, and 123c may be formed in the form of metal beads having magnetism. Accordingly, in the case of the computed tomography, the positions and forms of the beads 123, 123a, 123b, and 123c may be identified by color (e.g., white) that is distinguished from a living body (light or dark gray) in images ranging from black to white expressed by hounsfield scale (HU) values. In this case, as illustrated in FIG. 3, three beads 123a, 123b, and 123c may be placed equidistantly in a triangular shape when viewed in a plan view with respect to the magnetic sensor 125 attached to the bottom of the circuit board 124, and may be arranged to achieve a certain height difference with respect to the magnetic sensor 125. In order to reduce the magnetic interference, the three beads 123a, 123b, and 123c may be arranged on a surface of the housing 121 or case that protects the sensors 120 and 120' (see FIG. 8). Since a plane and coordinate axis are generated by these beads 123, 123a, and 123c, the position of the sensor may be detected or registration may be performed by comparing the coordinate axis before and after the procedure.

When the sensor 120 is directly attached to teeth, in order to improve adhesion to the teeth, shapes such as irregularities or holes may be formed on the housing 121 to increase a contact area.

As illustrated in FGIS. 5 to 7, the sensor 120 is arranged on the jig 130 that is approximately 'n' shape. The jig 130 includes a first side wall part 131 and a second side wall part 132 that are spaced apart from each other and a head part 133 connecting between the first side wall part 131 and the second side wall part 132. To fix the jig 130 to the teeth, an adhesive means 150 (see FIG. 1) may be used. The adhesive means 150 may be in the form of putty containing a dental adhesive.

Slots 135 and 136 may be formed in the first side wall part 131 and the second side wall part 132 through which the adhesive means 150 may seep out. These slots 135 and 136 are advantageous in terms of fixing force because they increase a bonding area between the teeth and an inner surface of the jig 130 when the adhesive means 150 is used. The slots 135 and 136 may extend parallel to a direction in which the jig 130 is pressed against the teeth. Reinforcement ribs 134 may be formed on edges of the first side wall part 131 and the second side wall part 132 to improve rigidity and make it easier to hold and use.

The head part 133 is formed with a plurality of through holes 137 and 138 through which the adhesive means 150 may seep out. The through holes 137 and 138 also increase a fixing force by increasing the bonding area between the teeth and the inner surface of the jig 130.

The housing 121 of the jig 130 or the sensor 120 may be made of a non-magnetic material such as polycarbonate so that the sensor 120 is not affected by the electromagnetic field.

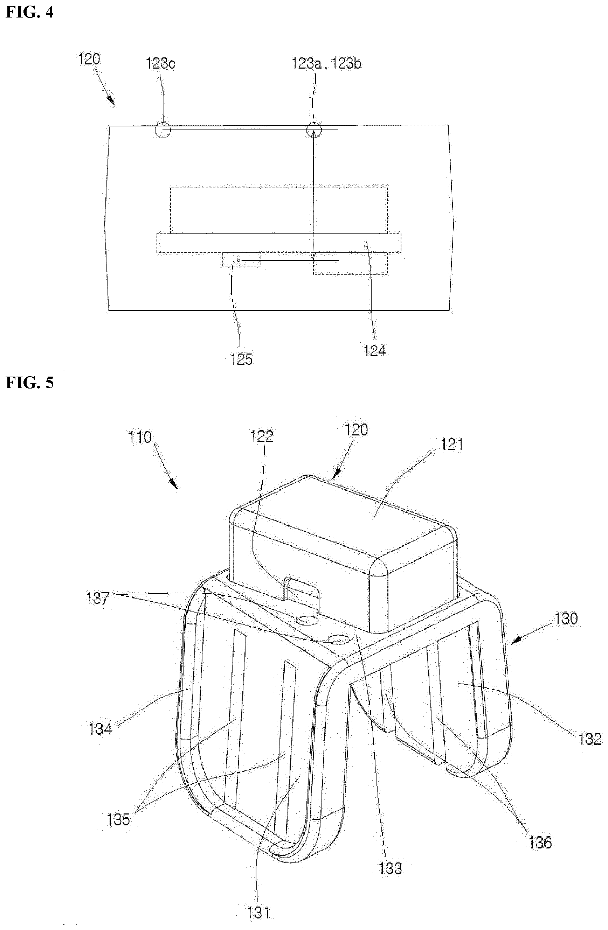

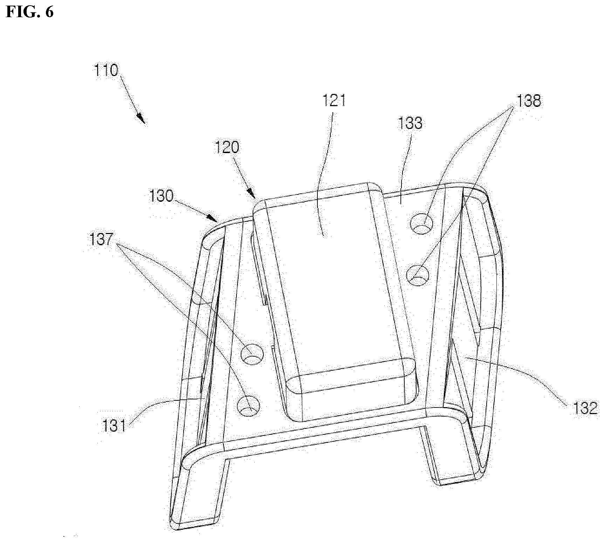

As illustrated in FIGS. 5 to 7, in the detection unit 110 of this example, the sensor 120 is arranged on the head part 133 of the jig 130 (horizontal jig).

FIGS. 8 to 10 illustrate a detection unit 110' having different types of jigs 130'. Even in this example, the jig 130' is common in that it includes a first side wall part 131', a second side wall part 132', and a head part 133' connecting between the first side wall part 131' and the second side wall part 132', and includes a slot 136', a through hole 137', and a reinforcement rib 134' as a shape to improve adhesion. On the other hand, in the detection unit 110' of this example, unlike the previous example, the sensor 120' is arranged on the first side wall part 131' (vertical jig).

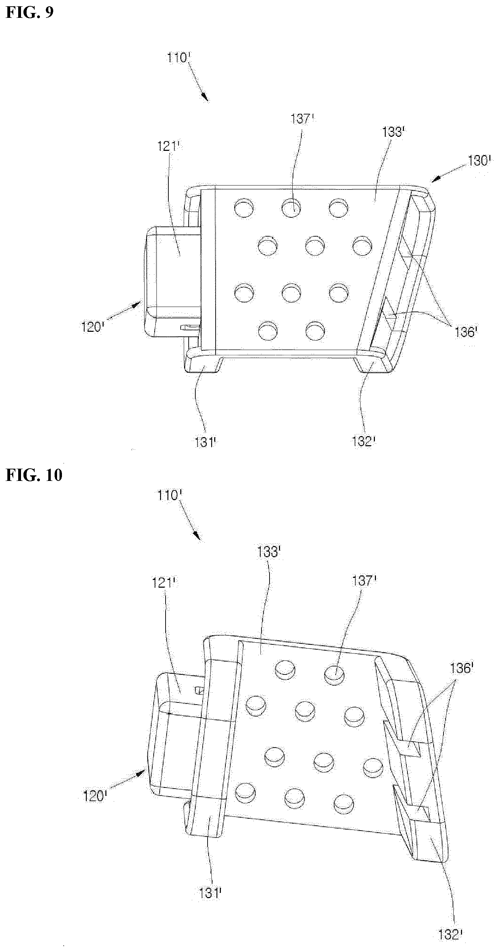

In this way, the detection units 110 and 110' may be classified into a plurality of types depending on the position or direction of the sensors 120 and 120' with respect to the jigs 130 and 130'. In addition, as illustrated in FIGS. 5 to 7, when the first side wall part 131 and the second side wall part 132 were parallel, and as illustrated in FIGS. 8 and 9, the first side wall part 131' and the second side wall part 132' may not be parallel but may be formed to be narrower toward one side.

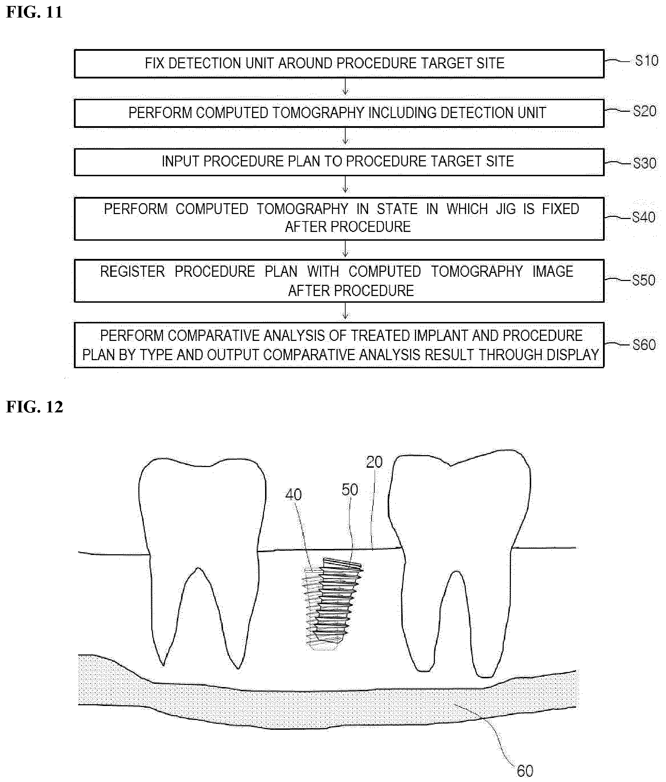

FIG. 11 is a flowchart for describing a method of analyzing implant placement results using the motion tracking system for dental implantation related to the present invention. In order to analyze whether the implant has been accurately placed, first, an appropriate one of the above-described detection units 110 and 110' is fixed around the procedure target site 20 (S10). The detection units 110 and 110' are provided with the sensors 120 and 120', thereby detecting changes in the position or direction of the procedure target site 20.

After fixing the detection units 110 and 110' to the teeth of the patient 10 or the like, a CT scan is performed on the detection units 110 and 110' along with the procedure target site 20 using the computed tomography device 170 (S20).

After obtaining the first image including the detection units 110 and 110' by the computed tomography, the operator makes a plan including the position (including depth and horizontal position) and direction of the implant to be placed and inputs the procedure plan through the user interface (S30). The control unit 180 may be configured to store the procedure plan and output the stored procedure plan through the display 160.

According to the procedure plan, the operator performs the implant placement procedure. In this case, during the procedure, the sensors 120 and 120' included in the detection units 110 and 110' may be configured to provide detection information so that the exact position or direction relationship between the procedure target site 20 and the handpiece held by the operator may be understood in real time.

After the procedure is completed, capturing is performed with the detection units 110 and 110' fixed using the computed tomography device 170 to analyze the placed implant (S40). The second image thus obtained also includes the beads 123a, 123b, and 123c, and the coordinate axis and plane of the beads 123a, 123b, and 123c in the second image are registered with the beads 123a, 123b, and 123c in the first image (S50). As a result, as illustrated in FIG. 12, the comparative analysis between the implant 40 according to the procedure plan and the post-procedure implant 50 is possible.

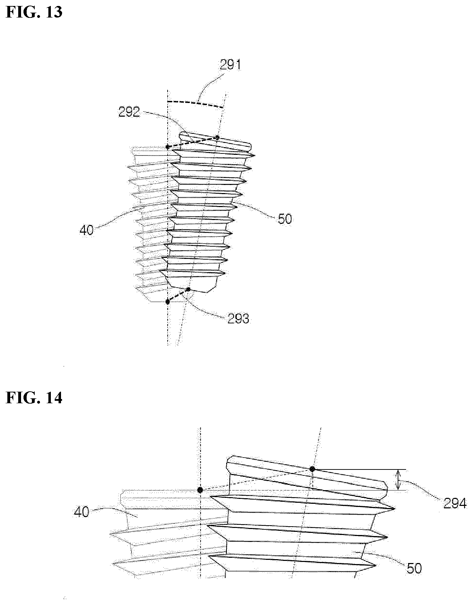

Next, the comparison between the implant 40 according to the procedure plan included in the second image and the post-procedure implant 50 is performed for each type and output through the display 160 (S60). FIGS. 13 and 14 are diagrams illustrating an appearance of an implant before and after placement for displaying measurement targets for each analysis type in relation to the present invention. Both the implant 40 in the procedure plan and the post-placement implant 50 have a three-dimensional position and direction by the computed tomography, and the accurate value may be obtained from the reference point using the detection units 110 and 110'. The analysis types of the implant placement results may include the following four types.

### (1) Angular Deviation

A three-dimensional angle 291 of the central axis of the procedure planned implant 40 and the central axis of the post-placement implant 50 may be measured, and the analysis results may be displayed or stored on the screen to check the difference from the procedure plan.

### (2) Signed Depth

A vertical distance 294 of the upper end of the central axis of the procedure planned implant 40 and the upper end of the central axis of the post-placement implant 50 may be measured, and the analysis results may be displayed or stored on the screen to check the difference from the procedure plan. This distance 294 may be reference information on how deeper or shallower the implant is placed than the procedure plan.

### (3) Coronal Deviation

A three-dimensional distance 292 of the upper end of the central axis of the procedure planned implant 40 and the upper end of the central axis of the post-placement implant 50 may be measured, and the analysis results may be displayed or stored on the screen to check the difference from the procedure plan.

### (4) Apical Deviation

A three-dimensional distance 293 of a lower end of the central axis of the procedure planned implant 40 and a lower end of the central axis of the post-placement implant 50 may be measured, and the analysis results may be displayed or stored on the screen to check the difference from the procedure plan.

In this way, it is possible to check the state of the dental implantation in detail and accurately through the provided comparison between the implant 40 in the procedure plan and the post-placement implant 50 for each type of analysis.

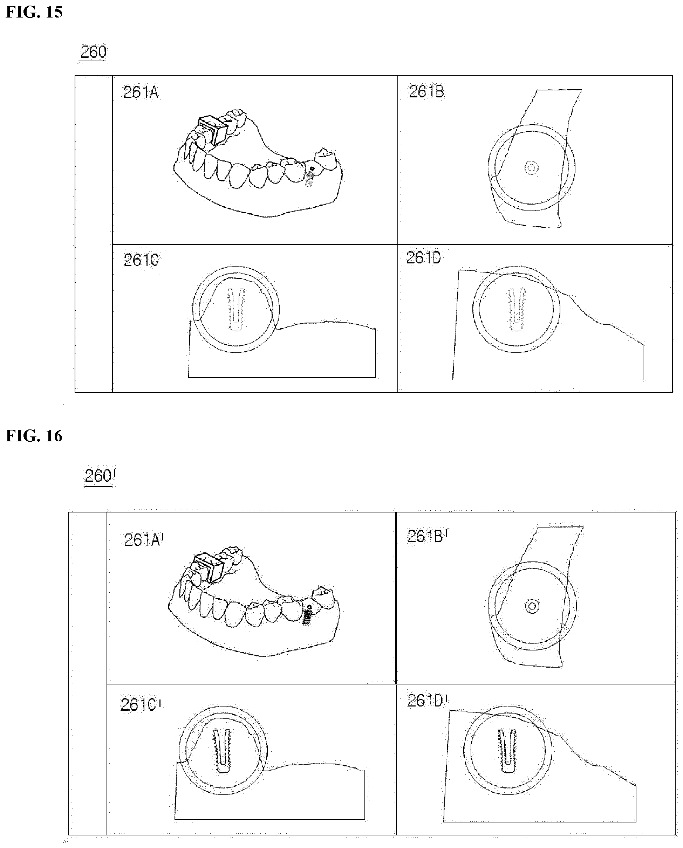

FIGS. 15 and 16 are examples related to the present invention, and are screens illustrating before and after placement displayed on the display, respectively.

In this example, the screens 260 and 260' are configured so that the plan before the procedure and the state of the implant after the procedure can be individually checked through several split screens 261A, 261A', 261B, 261B', 261C, 261C', 261D, and 261D'. In addition, the discrepancy in position or direction between the implant 40 according to the placement plan and the post-placement implant 50 can be presented through the distance or angle from the perspective of the axial, sagittal, and coronal planes on the screen all at once or individually. In this way, the computed tomography is performed before and after placement with the detection units 110 and 110' fixed, and the image registration is performed to compare the procedure planned implant 40 and the post-procedure implant 50 to obtain accurate measurement and analysis results for each type of analysis. When the implant placement training or procedure testing is performed, the objective and accurate check is possible during evaluation and verification before and after placement.

The control unit 180 is configured to calculate the position and change of the sensor 120 using the signal from the sensor 120, and provide a control signal for the movement M2 of the hole guide 340 connected to the robot 300 accordingly. The robot 300 is provided with the hole guide 340 formed to guide the position and direction of the procedure tool 4, such as the handpiece 400, with respect to the procedure target site 20. The hole guide 340 is a type that may be attached to or detached from the end effector of the robot 300. As described later, the hole guide 340 guides the position and entry direction of the procedure tool 4, such as a drill tip, at the end portion, and tracks the movement M1 (see FIG. 1) of the patient 10 according to the operation of the robot 300 and even if there is a change in the posture or motion of the patient 10 during the procedure, the movement M2 (see FIG. 1) of the hole guide 340 is guided in response to this change to ensure accurate procedure guidance.

FIG. 17 is a diagram schematically illustrating the side view of the hole guide related to the present invention, FIGS. 18A to 18C are plan views exemplarily illustrating the hole guide of FIG. 17, and FIG. 19 is a diagram schematically illustrating an appearance in which the hole guide 340 is attached to an end effector 331 of the robot.

As illustrated in these drawings, the hole guide 340 includes a mounting part 341 for mounting on the end effector 331 of the robot 300 and a guide part 342 that is formed at the end portion of the mounting part 341 and provided with a hole. As illustrated in FIGS. 18A to 18C, the hole guides 340, 340', and 340" may be formed in various types to be selectable depending on a size of a fixture to be placed. For example, compared to a diameter of the hole 343 formed in the guide part 342 of the hole guide 340 in FIG. 18A, the hole 343' formed in the guide part 342' of the hole guide 340' in FIG. 18B and the hole 343" formed in the guide part 342" of the hole guide 340" in FIG. 18C is sequentially made smaller. These hole guides 340, 340', and 340" may be formed in the same general form that can be applied to different patients. In addition, compared to the case where the hole guides 340, 340', and 340" may be used by making guides for each patient, it is possible to reduce the dental implantation stage and reduce the costs economically.

As illustrated in FIGS. 17 to 19, the guide parts 342, 342', and 342" may be formed in the form of a sleeve to guide the entry direction of the operator's procedure tools. The guide parts 342, 342', and 342" may include a lamp or display element that may provide visual indication for guidance during a separate procedure or a warning of incorrect positioning. To this end, the handpiece 400 and the like may be configured to detect a magnetic field from the base 101 and track the exact position of the handpiece 400.

FIG. 20 is a flowchart for describing a robot guide method.

First, after a CT image of an oral cavity is captured, an operator identifies the oral cavity structure based on the CT image, and establishes an implant placement plan, which is received by the control unit (S310). The placement plan may be information related to the position or direction for placing the implant 40, which may be input through a user interface for the display 160 and stored through the control unit 180.

Next, when the operator selects the hole guide 340 according to the size of the planned implant 40 and fastens the hole guide 340 to the end effector 331, the control unit 180 checks and receives information on the fastened hole guide 340 (S320).

To guide the dental implantation, the sensor 120 for tracking the motion of the patient is installed in an oral cavity of a patient and sets a reference position. As a specific method, the position and calculation operation of the hole guide 340 according to the motion of the patient are performed in a guide program. The end effector 331 of the robot 300 is arranged so that the position and direction of the implant placement plan and the center of the hole guide 340 are in the same position and direction in reality. Even if the motion of the patient 10 continues, after checking whether a guide function is maintained, if there are no abnormalities, the implant placement procedure starts. In this case, the center of the guide part 342 of the hole guide 340 is a portion where the implant 50 will be positioned, and the operator determines the hole guide 340 in real time and proceeds with the procedure. In this process, the results detected by the sensor 120 are received (S330), and the robot 300 is controlled to move the hole guide 340 in real time according to the change in position and direction according to the motion of the patient (S340 and S350). When the implant placement is completed, the hole guide 340 is moved to its original position (S360), and the implant placement procedure is ended.

By this method, the operator may adjust the exact position and direction of the procedure tool in real time regardless of the movement or motion of the patient through the robot guide.

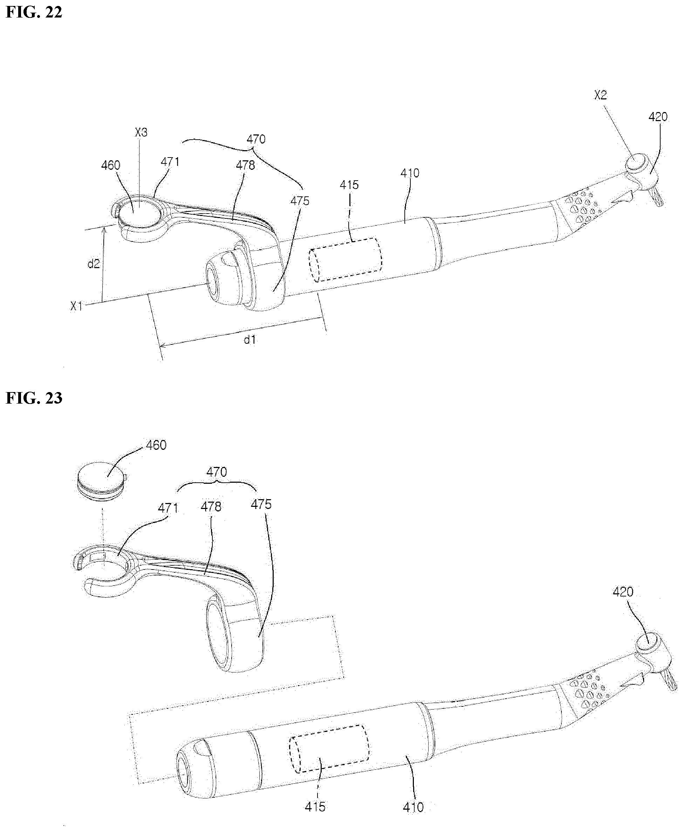

FIG. 21 is a diagram conceptually illustrating the motion tracking system for dental implantation to which a handpiece device capable of tracking a position is applied according to an example related to the present invention, FIG. 22 is a perspective view of the handpiece device capable of tracking the position related to the present invention, and FIG. 23 is an exploded perspective view of the handpiece device capable of tracking the position of FIG. 22.

The handpiece device 400 capable of tracking the position includes a body part 410 with a driving element 415 such as a DC motor or an air motor for various operations in the dental implantation stage. A tool mounting part 420 for mounting a tool 425 such as a drill tip is provided at a tip of the body part 410. The body part 410 may be electrically connected to the control unit 180 to control the operation or speed of the driving element 415, and may be connected to a water supply 60 through a hose to spray water.

A sensor module 450 related to the present invention is mounted on a rear end of the body part 410. The sensor module 450 includes a sensor unit 460 that is spatially spaced apart from the driving element 415, and a spacing jig 470 that supports the sensor unit 460 against the body part 410. The spacing jig 470 includes a support part 471 for fixing the sensor unit 460, a coupling part 475 coupled to the body part 410, and a space extension part 478 connecting between the support part 471 and the coupling part 475.

The sensor unit 460 may include a magnetic field sensor. When the sensor unit 460 detects the magnetic field from the base 101 and transmits the detection result to the control unit 180, the control unit 180 may be configured to identify and track the position and posture of the handpiece device 400 through the calculation and visually display the identified and tracked position and posture through the display 160 or provide necessary feedback or warnings to the operator.

As illustrated in FIG. 22, the body part 410 may be formed to extend in a first axis X1 direction, and the tool mounting part 420 may be formed so that the tool 425 faces a second axis X2 direction. The sensor unit 460 may have a separate third axis X3 for setting the reference. In this case, the space extension part 478 has a length and direction so that the sensor unit 460 is spaced apart from the driving element 415 by a first offset d1 along the first axis X1 direction and spaced by a second offset d2 with respect to the first axis X1. As a result, the influence of electromagnetic interference on the driving element 415 may be significantly reduced, and there is no conflict with the arrangement of connection elements such as cables connected to the body part 410.

As an embodiment, the sensor unit 460 may be arranged on a plane formed by the first axis X1 and the second axis X2. This means that the spaced arrangement of the sensor unit 460 does not cause inconvenience to the operator holding the body part 410. As another example, the coupling part 475 may be coupled while rotating by an angle selected with respect to the body part 410 so that the sensor unit 460 forms a predetermined angle with respect to the plane formed by the first axis X1 and the second axis X2. When the angle with respect to the body part 410 of the spacing jig 470 is changed, before starting the procedure, it is possible to adjust to the changed reference point by setting the reference point. As illustrated in FIG. 23, the coupling part 475 may be formed in the form of a ring that is inserted into the body part 410.

As illustrated in FIG. 23, the support part 471 may be formed in a form with the rear open so that the cable connecting the sensor unit 460 may be connected from the rear when viewed from the body part 410. In terms of manufacturing, the spacing jig 470 may simplify manufacturing and reduce costs by integrally forming the support part 471, the coupling part 475, and the space extension part 478. In this case, it is preferable that the housing of the body part 410 together with the support part 471, the coupling part 475, and the space extension part 478 that constitute the spacing jig 470 are all made of non-magnetic materials (e.g., resin, titanium) etc.

In this way, the sensor unit 460 is sufficiently spaced apart from the driving element 415 by the spacing jig 470, thereby minimizing the interference caused by the electromagnetic field and avoiding hindering the operation efficiency of the operator who holds the body part 410.

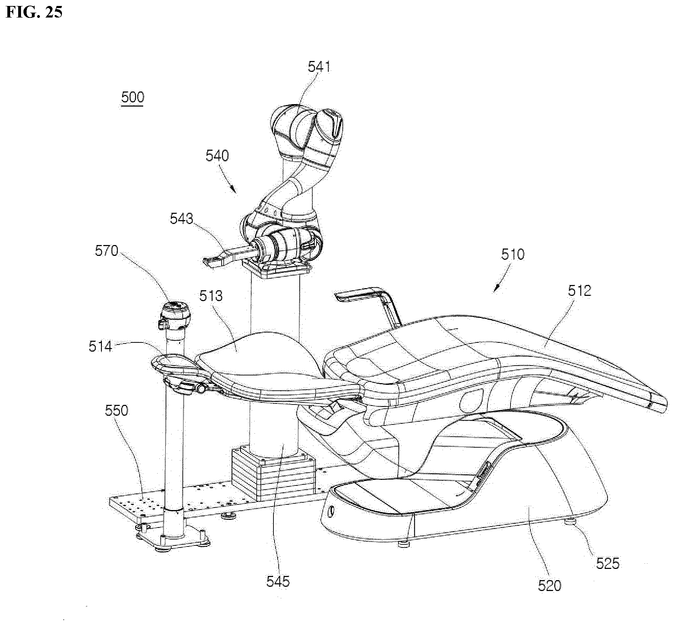

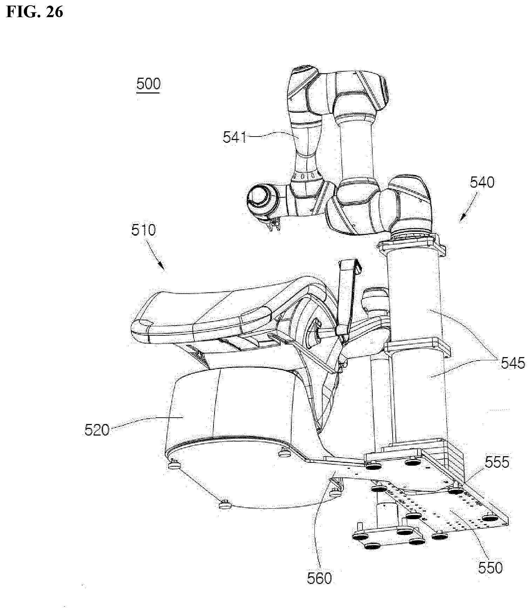

FIG. 24 is a perspective view of an integrated dental chair system 500 related to the present invention, FIG. 25 is a perspective view illustrating the dental chair system 500 of FIG. 24 viewed from another side, and FIG. 26 is a perspective view illustrating the dental chair system 500 of FIG. 24 viewed from bottom.

As illustrated in these drawings, the dental chair system 500 related to the present invention includes a chair part 510 formed to adjust the patient's sitting or lying posture and a robot 540 for assisting the operator's procedure. In these drawings, various functional units that may be added for procedures or treatments, such as a handpiece, an air or water supply device, and a suction device, may also be provided, but are omitted for the gist of the present embodiment.

The chair part 510 is supported on the lower chair base 520, and may include a chair body 512, a backrest 513, and a headrest 514 that may allow a patient to sit or lie down at any angle or adjust an angle, and driving devices (not illustrated) for driving the chair body 512, the backrest 513, and the headrest 514. A plurality of first ground parts 525 are provided on a lower surface of the chair base 520 to support the chair part 510 on the ground.

One side of the chair part 510 is mounted with the robot 540 that may assist the procedure by moving an arm 541 based on the position with respect to the procedure target site of the patient. A robot base 550 formed to support the robot 540 is arranged at a lower portion of the robot 540. The lower portion of the robot base 550 includes a plurality of second ground parts 555 to be supported on the ground. A first spacer 545 may be provided between the robot 540 and the robot base 550 to adjust the position of the robot 540 during installation. Various end effectors 543 may be mounted at an end portion of the arm 541 depending on the stage assisting the procedure.

The chair base 520 and the robot base 550 are connected by a connection part 560, and thus the chair part 510 and the robot 540 are formed integrally. The connection part 560 extends from the lower portion of the chair base 520 and is connected to the robot base 550. According to FIGS. 24 to 26, the connection part 560 is located at a lower end of the chair base 520. The connection part 560 may include a fastening part 565 for fastening and coupling the chair base 520 and the robot base 550. The connection part 560 connects the chair base 520 and the robot base 550 and fixes their positional relationship, making it easy to set a reference position for the robot 540 to operate, and may reduce the time required for setting up before the procedure, which may occur due to independently positioning the chair part 510 and the robot 540.

A base 570, which may generate an energy field that is a detection target of the sensor unit outside a patient, is installed on one side of the chair part 510. The base 570 may include a magnetic field generation unit capable of generating a magnetic field.

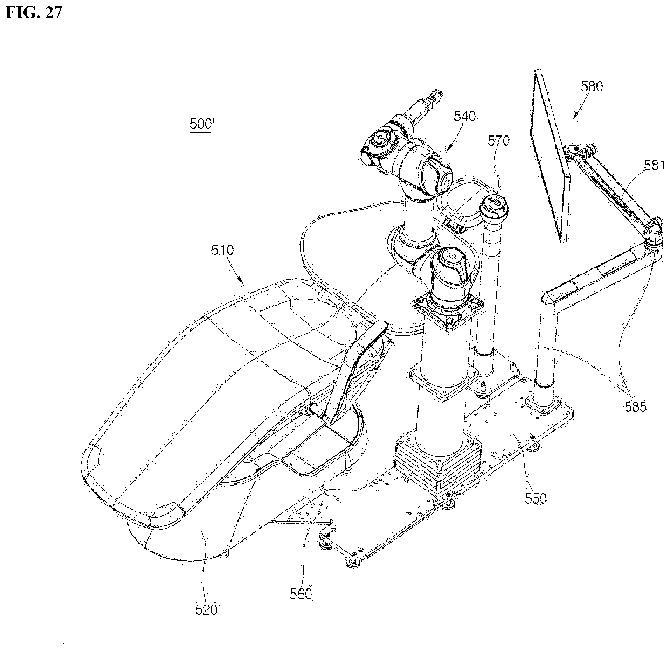

FIG. 27 is a perspective view of a dental chair system 500' according to another example related to the present invention. In the dental chair system 500' of this example, the chair part 510 supported by the chair base 520 and the robot 540 supported by the robot base 550 are integrated through the connection part 560. In addition, the dental chair system 500' also includes a display 580 installed on the robot base 550. The display 580 is configured to display information about the erection position and direction or the progress direction of the placement when the operator places the implant.

One end of the display 580 is fixed to the robot base 550 and the other end is coupled to the display device 580, and thus, the display device 580 is supported by a balance arm 581 so that its position and posture can be changed. In this case, the balance arm 581 may include one or a plurality of link members and spring elements. This balance arm 581 may be configured to adjust its position or height by at least one second spacer 585. The display device 580 supported on the balance arm 581 may provide flexibility for movement and reduce physical fatigue of operators occurring during the procedure.

According to the integrated dental chair system according to the present embodiment, the chair part on which the patient sits and the robot that assists the procedure are integrated by the connection part connecting between the chair base and the robot base, so there is no need for a bulky robot base, and installation in the operating room is simple. In addition, by establishing the patient's approximate position and the surgical environment to be guided by the robot during the initial installation through the integrated structure, there is no need to re-establish the surgical environment each time the surgery is performed, thereby shortening the dental implantation time and reducing physical fatigue of nurses and surgeons.

According to a motion tracking system for dental implantation of the present invention, a detection unit is attached to teeth spaced from a procedure target site by an adhesive means and is classified into a plurality of types depending on a position or direction of a sensor, and an implant guide device can be used for common purposes without needing to be manufactured for each patient, thereby saving time and materials required for manufacturing and increasing productivity. In addition, compared to the conventional example of having to bite a large bite, inconvenience can be eliminated, and a motion of a patient can be tracked in real time by detecting a magnetic field, thereby providing intuitive and accurate procedure information.

According to another example related to the present invention, by reflecting a real-time motion tracking function by a detection unit having a metal bead in a computed tomography image, it is possible to accurately identify a procedure situation in comparison with a procedure plan.

According to another example related to the present invention, by using computed tomography images before and after procedure and a detection unit having a metal bead, it is possible to provide comparative analysis results before and after the procedure. For example, when implant placement training or procedure testing is conducted, it is possible to implement objective and accurate confirmation during evaluation and verification before and after placement. In addition, it is possible to improve the reliability of the procedure by sharing the placement state or results with a patient.

The motion tracking system for dental implantation described above is not limited to the configuration and method of the described embodiments. All or some of the respective exemplary embodiments may be selectively combined with each other so that the above-mentioned exemplary embodiments may be variously modified to substitutable equivalents.

## Claims

1. A motion tracking system for dental implantation, comprising:
a base that is formed to generate an electromagnetic field;
a detection unit that is attached around a procedure target site and includes a sensor detecting the electromagnetic field generated from the base; and
a control unit that receives a measurement signal from the sensor, calculates a variation in a position and direction of the procedure target site, and outputs the calculated amount of change through a display,
wherein the detection unit is formed to be attached to teeth spaced from the procedure target site in a dental arch of a patient using an adhesive means preferably including putty containing an adhesive, and is classified into a plurality of types depending on the position or direction of the sensor.

2. The motion tracking system of claim 1, wherein the detection unit includes a jig, preferably made of a non-magnetic material, that has an inner side surface surrounding the teeth and outer side surface formed to have the sensor arranged thereon.

3. The motion tracking system of claim 2, wherein the jig includes a first side wall part, a second side wall part, and a head part connecting between the first side wall part and the second side wall part, and
the sensor is positioned in at least one selected from the first side wall part, the second side wall part, and the head part depending on the type of the detection unit,
wherein at least one of the first side wall part and the second side wall part includes a preferably slot through which the adhesive means seeps out.

4. The motion tracking system of claim 3, further comprising reinforcement ribs that are formed along edges of the first side wall part and the second side wall part, and/or
a plurality of through holes that are formed in the head part and formed to allow the adhesive means to seep out,
wherein the type of the detection unit preferably includes a form in which the first side wall part and the second side wall part are parallel and is not parallel..

5. The motion tracking system of one of the proceeding claims, wherein the sensor includes a housing that is formed to be directly attached to teeth by the adhesive means.

6. The motion tracking system of one of the proceeding claims, further comprising a computed tomography device that is configured to image the procedure target site and the detection unit,
wherein the sensor includes a plurality of metal beads having magnetism arranged so that the sensor position is detected in an image captured by the computed tomography device.

7. The motion tracking system of claim 6, wherein the control unit is configured to reflect and output the variation in the position and direction of the procedure target site calculated through the measurement signal of the sensor when a motion of a patient occurs in a position and direction of an implant planned for the computed tomography image while the detection unit is fixed, and/or
wherein the control unit is formed to register a procedure plan with a second image based on the detection unit from a first image obtained by computed tomography while the detection unit is fixed, a procedure plan including a position and direction of an implant to be placed in the first image, and the second image obtained by the computed tomography while the detection unit is fixed after the procedure, and provide a comparison result of the implant through the display after the procedure included in the second image and the procedure plan.

8. The motion tracking system of one of the proceeding claims, further comprising:
a robot that is formed to be operated by a control signal of the control unit according to a procedure plan based on a calculation result of the control unit; and
a hole guide that is formed to be detachable from the robot and formed to guide a position and direction of a procedure tool for the procedure target site.

9. The motion tracking system of claim 8, wherein the hole guide includes:
a mounting part that is mounted on an end effector of the robot; and
a guide part that is formed at an end portion of the mounting part and formed with a hole.

10. The motion tracking system of claim 9, wherein the guide part is formed in a form of a sleeve to guide an entry direction of procedure tools of an operator.

11. The motion tracking system of one of the proceeding claims, further comprising:
a body part that is provided with a driving element generating the electromagnetic field;
a tool mounting part that is formed to enable a tool to be mounted on a tip of the body part; and
a sensor unit that is formed to detect a position of the tool arranged in space to track the position of the tool; and
a spacing jig that has one end formed with a support part for fixing the sensor unit and the other end formed with a coupling part coupled to the body part, and is formed to maintain the sensor unit spatially spaced from the driving element,
wherein the coupling part is preferably formed in a form of a ring that is inserted into the body part.

12. The motion tracking system of claim 11, wherein the spacing jig includes a space extension part connecting the support part and the coupling part, preferably having a direction and length so that the sensor unit is spaced apart from the driving element along the first axis X1 direction by a first offset d1 and is spaced apart from the first axis X1 by a second offset d2.

13. The motion tracking system of claim 12, wherein the body part extends in a first axis X1 direction, and the tool is formed to face in a second axis X2 direction, and the sensor unit is arranged on a plane formed by the first axis X1 and the second axis X2, and wherein the coupling part is preferably coupled to the body part in a rotated state by a selected angle with respect to the body part so that the sensor unit forms a predetermined angle with respect to the plane formed by the first axis X1 and the second axis X2.

14. The motion tracking system of one of the proceeding claims, further comprising:
a chair part that is formed to adjust a posture of a patient;
a chair base that supports the chair part and that preferably includes a plurality of first ground parts for being supported on a ground;
a robot that is formed to assist procedure by moving an arm with respect to the procedure target site of the patient based on the position;
a robot base that is formed to support the robot and that preferably includes a plurality of second ground parts for being supported on the ground;
a connection part that connects the chair base and the robot base to integrate the chair portion and the robot, and
optionally a first spacer that is arranged between the robot and the robot base.

15. The motion tracking system of claim 14, wherein the display is supported by a balance arm that has one end fixed to the robot base and the other end coupled to the display so that the display changes its position and posture.
